# EUROPÄISCHE PATENTANMELDUNG

(11) **EP 2 078 535 A1**
(43) Veröffentlichungstag der Anmeldung: **15.07.2009**
(21) Anmeldenummer: 09000212.2
(22) Anmeldetag: 09.01.2009
(51) Int. Cl.: A61M 37/00

(54) **Implantatspritze**

(30) Priorität: 10.01.2008 DE 102008003885; 30.01.2008 DE 102008006602
(71) Anmelder: Schmidt, Ulrich, 63571 Gelnhausen (DE)
(72) Erfinder: Schmidt, Ulrich, 63571 Gelnhausen (DE)
(74) Vertreter: Jochem, Bernd

(57) **Zusammenfassung**

Die Implantatspritze hat eine an einem Griff (12) gelagerte Kanüle (14) und einen in dieser angeordneten Ausstoßer (16) für die transkutane Einführung von z. B. Arznei- oder Schmerzmittel enthaltenden Festkörpern (10) durch die Kanüle (14) in den menschlichen oder tierischen Körper. Um den Festkörper (10) auch unter einem spitzen Winkel zur Hautoberfläche möglichst genau und schmerzfrei implantieren zu können, ist vorgesehen, dass die Kanüle (14) und der Ausstoßer (16) in einer Relativstellung am Griff (12) festlegbar sind, in der sich das vordere Ende des Ausstoßers (16) in einem bestimmten, für die Aufnahme eines oder mehrerer Festkörper (10) in der Kanüle (14) ausreichenden Abstand von deren Spitze befindet. Wenn bei dem Einstechvorgang die Spitze der Kanüle und damit auch der oder die Festkörper (10) ihre vorbestimmten Positionen im menschlichen oder tierischen Körper erreicht haben, ist nach dem Lösen einer Sperre (30) die Kanüle (14) durch die Kraft einer in einem hohlzylindrischen Griff (12) aufgenommenen Feder (18) oder alternativ mittels eines mit der Kanüle (14) verbundenen Schiebers von Hand relativ zum Griff (12) und dem Ausstoßer (16) bis hinter dessen vorderes Ende zurückziehbar.

## Beschreibung

Die Erfindung betrifft eine Implantatspritze mit einer an einem Griff gelagerten Kanüle und einem in dieser angeordneten Ausstoßer für die transkutane Einführung von z. B. Arznei- oder Schmerzmittel enthaltenden Festkörpern durch die Kanüle in den menschlichen oder tierischen Körper, wobei die Kanüle und der Ausstoßer in einer Relativstellung am Griff festlegbar sind, in der sich das vordere Ende des Ausstoßers in einem bestimmten, für die Aufnahme eines oder mehrerer Festkörper in der Kanüle ausreichenden Abstand von deren Spitze befindet, und dass die Kanüle nach dem Lösen einer Sperre relativ zum Griff und dem Ausstoßer bis hinter dessen vorderes Ende zurückziehbar ist.

Es gibt Arzneimittel und Schmerzmittel, die als Festkörper, z. B. kleine Zylinder oder Kugeln, in die Fettschicht oder ein anderes Gewebe unter der Haut eingebracht werden und dort ihre Wirkstoffe über einen sehr langen Zeitraum, ggf. über mehrere Monate, kontinuierlich abgeben. Diese und'zukünftig auch andere kleine Festkörper, z. B. für diagnostische und Identifikationszwecke, können am einfachsten mit einer Implantatspritze appliziert werden.

In einfacher Ausführung hat eine Implantatspritze, ähnlich wie eine Injektionsspritze, eine fest mit einem Griff verbundene Kanüle und einen Ausstoßer, der hier die Form eines längsverschieblich in der Kanüle geführten Stabs hat. Auch das hintere Ende des Stabs ist mit einem Griff versehen, so dass er nach dem Ein- oder Durchstechen der Haut zum Ausstoßen eines in der Kanüle aufgenommenen Implantats gegen die Kraft einer Rückstellfeder vorgeschoben werden kann, bis sein vorderes Ende nach vorne aus der Kanüle herausragt. Nach dem Ausstoßen des Implantats aus der Kanüle wird sie von Hand aus der Haut herausgezogen. Bei einer anderen bekannten Implantatspritze erfolgt das Herausziehen der Kanüle aus der Haut dadurch, dass nach dem Ausstoßen eines Implantats aus der Kanüle mittels des Stabs eine durch eine Feder belastete, die Kanüle umgebende Hülse nach vorne gegen die Haut gestoßen wird, so dass die Kanüle aus der Haut und mit ihrer Spitze in eine ungefährliche Stellung in die Hülse gezogen wird.

Derartige Implantatspritzen haben den Nachteil, dass ihre Handhabung beim Ausstoßen des Implantats schwierig ist. Einerseits muss die mit ihrer Spitze an eine bestimmte Stelle im Körper gebrachte Kanüle dort freihändig festgehalten werden, während die Ausstoßer-Stange vorsichtig vorgeschoben wird, um das Implantat aus der Kanüle und dann mit größerem Vorschubwiderstand vor dieser in das Gewebe zu stoßen. Dieser Vorgang ist oft schmerzhaft und kann zum Zerbrechen des Implantats führen. Außerdem ist es wegen der schwierigen Koordination der Handbewegungen zum Halten der Kanüle bei gleichzeitigem Vorschieben der Ausstoßer-Stange kaum möglich, das Implantat genau an der richtigen Stelle zu deponieren.

Aus der US-A-4105030 ist auch bereits ein Implantationsapparat für die Tiermedizin bekannt, der die Form einer Pistole hat, wobei an der Stelle des Laufs eine Schlittenführung mit zwei Führungsbahnen und zwei Führungsstangen, auf denen vier Schraubenfedern sitzen, vorhanden ist, längs der ein eine nach vorne vorstehende Kanüle tragender Schlitten verschieblich ist. Durch eine Bohrung in dem Schlitten erstreckt sich ein am hinteren Ende des Pistolengehäuses lösbar befestigter Ausstoßerstab in die Kanüle. Nach dem Durchstechen der Haut wird der am Pistolengriff verschwenkbar gelagerte, verriegelbare Abzugshebel betätigt und gibt den in seiner vorderen Endstellung unter der Vorspannung von zwei Federn gehaltenen Schlitten frei, so dass die Kanüle bis hinter die Vorderkante des Pistolengehäuses zurückgezogen wird, während das über diese Vorderkante vorstehende vordere Ende des Ausstoßerstabs das Implantat ortsfest hält. Der Apparat hat den Nachteil, dass er einen voluminösen, komplizierten und entsprechend teuren Aufbau hat und durch ein verhältnismäßig großflächiges vorderes Ende des Gehäuses in der Anwendung auf ein Einstechen senkrecht zur Oberfläche der Haut oder nur mit geringer Neigung beschränkt ist.

Von diesem Stand der Technik ausgehend, liegt der Erfindung die Aufgabe zugrunde, eine Implantatspritze der eingangs genannten Art zur Verfügung zu stellen, die einen so einfachen, kostengünstigen Aufbau hat, dass sie als Einwegspritze ausgeführt werden kann, und die sich auch sehr schräg in die Haut einstechen lässt, um ein Implantat angenähert parallel zur Hautoberfläche deponieren zu können.

Vorstehende Aufgabe wird bei der eingangs bezeichneten Implantatspritze dadurch gelöst, dass die Kanüle mittels eines in einem hohlzylindrischen Griff axial geführten Federkolbens durch die Kraft einer in dem zylindrischen Hohlraum des Griffs aufgenommenen, vorgespannten Feder zurückziehbar ist.

Die neue Implantatspritze ist nur wenig komplizierter als eine Injektionsspritze, der sie trotz unterschiedlicher Funktionsweise äußerlich ähnlich sieht. Daher fallen auch bei nur einmaligem Gebrauch ihre Kosten nicht ins Gewicht. Ein breites Anwendungsspektrum, darunter auch ein sehr flaches Einstechen in die Haut, ist eine weitere Gemeinsamkeit mit Injektionsspritzen.

Beim Einstechen der vorgeschlagenen Implantatspritze in die Haut lässt sich der hohlzylindrische Griff von Hand genau führen, während die Kanüle, der Ausstoßer und das unmittelbar hinter der Spitze der Kanüle durch den Ausstoßer axial gehaltene Implantat gemeinsam vorgeschoben werden, bis die Kanüle diejenige Stellung im Körper erreicht, in der sich das Implantat in der vorgesehenen Position befindet. Anschließend wird die Sperre zwischen der Kanüle und dem Griff gelöst und die Kanüle durch die zentrale Feder zurückgezogen, während der Ausstoßer gegen Zurückziehen gehalten wird, bis sich zumindest soviel von dem Implantat außerhalb der Kanüle befindet, dass es an der vorgesehenen Stelle liegen bleibt, wenn nachfolgend der Ausstoßer und die Kanüle weiter zurückgezogen werden. Für das Zurückziehen der Kanüle von dem positionierten Implantat braucht man nur eine minimale Kraft. Daher fällt in dieser Phase die Handhabung der Implantatspritze leicht und verursacht den Patienten auch keine Schmerzen.

In der bevorzugten praktischen Ausführung ist die Kanüle von einer Schrauben-Druckfeder umgeben, die im vorgespannten Zustand in dem zur Kanüle koaxialen, zylindrischen Hohlraum im Griff zwischen einem Federlager und dem mit dem hinteren Ende der Kanüle verbundenen Federkolben eingespannt ist, der bei vorgespannter Feder durch einen Riegel am Griff festlegbar und mittels eines an der Außenseite des Griffs angebrachten, auf den Riegel wirkenden Betätigungselements entriegelbar ist. Im Gegensatz zu der axial verschieblichen Kanüle ist in der bevorzugten praktischen Ausführung der Ausstoßer ein mit seinem hinteren Ende am hinteren Ende des Griffs befestigter, sich durch den Federkolben in die Kanüle erstreckender Stab, dessen vorderes Ende aus dem vorderen Ende des Griffs herausragt. Alternativ kann der Ausstoßer aber auch ein am vorderen Ende des Griffs über einen einseitig angeordneten, schmalen radialen Steg befestigter Stab sein, wenn die Kanüle mit einem Längsschlitz versehen ist, durch den sich der radiale Steg erstreckt.

Die neue Implantatspritze muss nicht notwendigerweise einen hohlzylindrischen Griff und darin einen Federkolben zum Zurückziehen der Kanüle haben. Alternativ kann auch ein flacher Griff mit einem mit der Kanüle verbundenen Schieber Verwendung finden, der mit einem Finger zurückgezogen wird.

Da bei der erfindungsgemäßen Implantatspritze das Implantat bis zum Zurückziehen der Kanüle vom Ausstoßer unmittelbar hinter deren Öffnung gehalten wird, ist es besonders wichtig, Vorkehrungen zu treffen, dass das Implantat nicht vorzeitig aus der Kanüle fällt oder rutscht. Dieses Problem besteht aber auch bei anderen Implantatspritzen. Als Abhilfe wird vorgeschlagen, dass am vorderen Ende des Griffs ein elastischer Draht befestigt oder eine elastische Zunge aus Kunststoff angebracht oder angeformt ist, dessen bzw. deren zu einer Öse oder einem Kopf gebogenes, freies Ende sich unmittelbar vor der Öffnung der in ihre vordere Endstellung vorgeschobenen Kanüle befindet und beim Einstechen der Kanüle in die Haut auslenkbar ist.

Nachfolgend werden Ausführungsbeispiele der Erfindung anhand der beigefügten Zeichnung näher erläutert. Es zeigen:
- Fig. 1: eine perspektivische Ansicht schräg von vorne eines ersten Ausführungsbeispiels einer Implantatspritze im gesicherten Ausgangszustand;
- Fig. 2: die Implantatspritze nach Fig. 1 im Längsschnitt;
- Fig. 3: den vorderen Bereich des Längsschnitts nach Fig. 2 in größerem Maßstab;
- Fig. 4: in einer Ansicht entsprechend Fig. 1 die Implantatspritze im entsicherten Zustand;
- Fig. 5: im Längsschnitt gemäß Fig. 2 die Implantatspritze nach Freilegen des Implantats durch Zurückziehen der Kanüle;
- Fig. 6: eine perspektivische Darstellung der Einzelteile der Implantatspritze nach Fig. 1 bis 5;
- Fig. 7: eine perspektivische Ansicht eines zweiten Ausführungsbeispiels einer Implantatspritze im Ausgangszustand;
- Fig. 8: in einer Ansicht entsprechend Fig. 7 die Implantatspritze nach dem Freilegen des Implantats durch Zurückziehen der Kanüle;
- Fig. 9: eine perspektivische Ansicht der Einzelteile der Implantatspritze nach Fig. 7 und 8;
- Fig. 10: eine perspektivische Ansicht eines dritten Ausführungsbeispiels einer Implantatspritze im Ausgangszustand;
- Fig. 11: eine Seitenansicht der Implantatspritze nach Fig. 10 nach der Abnahme einer vorderen Schutzhülse und eines Sicherungskeils;
- Fig. 12: eine perspektivische Ansicht der Implantatspritze nach Fig. 10 und 11 mit aufgeschnittenem Griff;
- Fig. 13: die Implantatspritze nach Fig. 10 und 11 in einer der Fig. 12 entsprechenden Ansicht nach dem Zurückziehen der Kanuüle, und
- Fig. 14: eine perspektivische Ansicht der Einzelteile der Implantatspritze nach Fig. 10 bis 13.

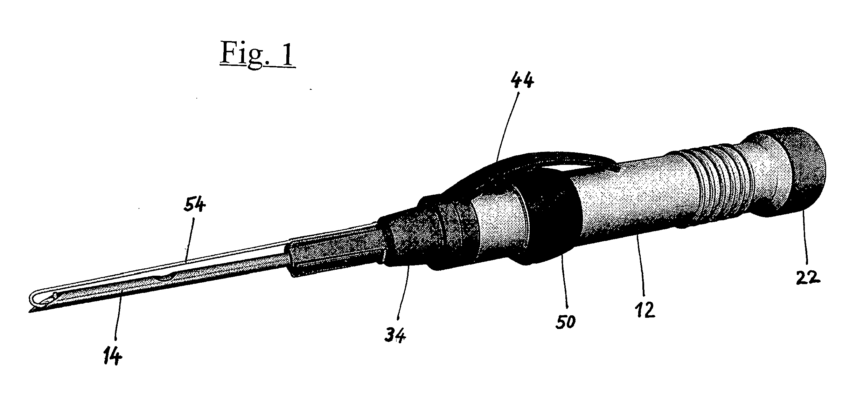

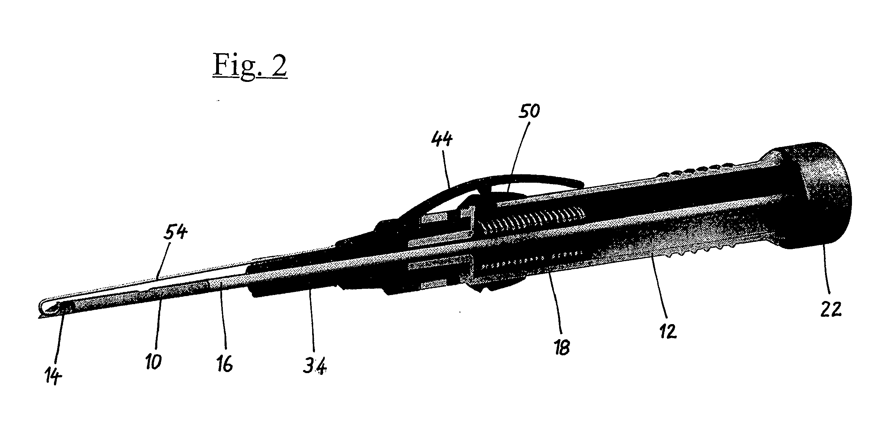

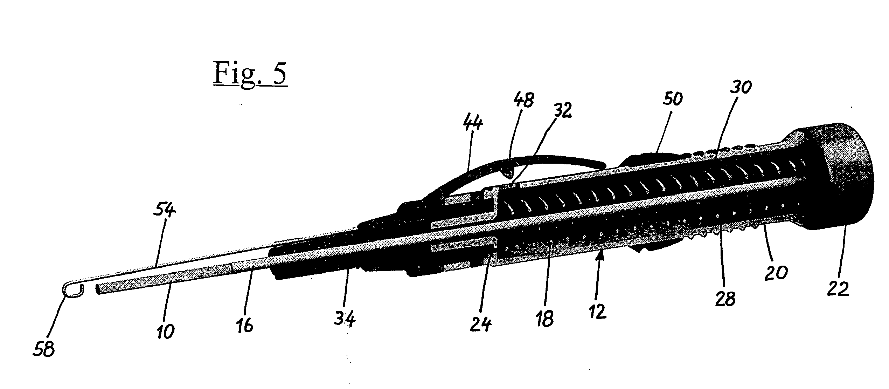

Die in der Zeichnung gezeigten Implantatspritzen dienen dazu, ein als zylindrischer Festkörper 10 vorliegendes Arznei- oder Schmerzmittel durch die Haut hindurch in einen menschlichen oder tierischen Körper zu implantieren, z. B. an einer bestimmten Stelle im Fettgewebe zu positionieren. Zu diesem Zweck hat die in Fig. 1 bis 6 gezeigte Implantatspritze eine an einem hohlzylindrischen Griff 12 festlegbare Kanüle 14 zur Aufnahme des Festkörpers 10 und einen Ausstoßer 16 in Form einer sich von hinten in die Kanüle 14 erstreckenden Stange. Während die Stange 16 an ihrem hinteren Ende fest mit dem Griff 12 verbunden ist, wird die Kanüle 14 nach dem Durchstechen wenigstens der äußeren Hautschicht durch eine im zylindrischen Hohlraum des Griffs 12 aufgenommene, im Ausgangszustand vorgespannte Druckfeder 18 so weit in den Griff 12 zurückgezogen, dass der im Ausgangszustand im vorderen Endbereich der Kanüle 14 gelagerte und durch die Stange 16 abgestützte Festkörper 10 ganz oder wenigstens zum größten Teil freiliegt, so dass er seine Lage im Körper nicht mehr ändert, wenn anschließend die Implantatspritze wieder aus dem menschlichen oder tierischen Körper herausgezogen wird. Vorzugsweise wird die Kanüle 14 durch die Feder 18 so weit zurückgezogen, dass sich ihr angespitztes, offenes vorderes Ende hinter dem vorderen Ende der Stange 16, möglichst sogar im Inneren des Griffs 12 befindet. Dann liegt der wirkstoffhaltige Festkörper 10 völlig frei vor dem vorderen Ende der Stange 16, und es braucht nur noch diese aus der Haut herausgezogen zu werden.

Die beschriebene Implantation des Festkörpers 10 ist mit minimalen Schmerzen verbunden, weil die angespitzte Kanüle verhältnismäßig leicht in das von ihr angeschnittene Gewebe vordringt und durch den während der Penetration fest mit ihr verbundenen, vorne verjüngten zylindrischen Griff von Hand geradlinig und sicher an die vorgesehene Stelle im Körper geführt werden kann. Danach braucht der Griff 12 nur noch ruhig gehalten und die Verriegelung, welche die Druckfeder im vorgespannten Zustand gehalten hat, gelöst zu werden. Die dadurch ausgelöste Rückzugsbewegung der Kanüle 14 ist weder schmerzhaft noch ändert sich dadurch die bereits beim Einstechvorgang erreichte, vorbestimmte Lage des Festkörpers 10, denn dieser wird auch während der Rückzugsbewegung der Kanüle 14 durch die Stange 16 axial abgestützt und dadurch in seiner Position gehalten. Das Ausstoßen des Festkörpers 10 aus der Kanüle 14 durch die Stange 16 ist also in diesem Fall weder mit einem weiteren Vorschub in den menschlichen oder tierischen Körper hinein noch mit schwierig zu koordinierenden, gegenläufigen Bewegungen der Kanüle 14 und der Ausstoßerstange 16 verbunden, denn diese braucht nur festgehalten zu werden.

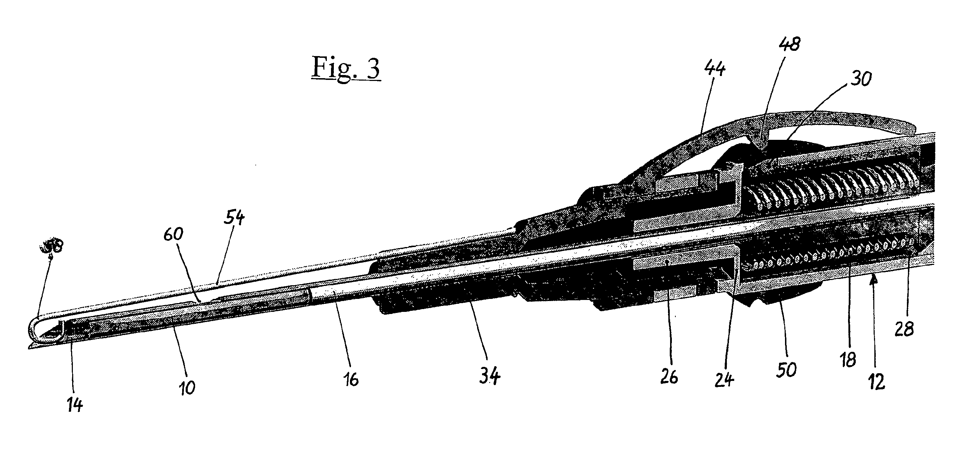

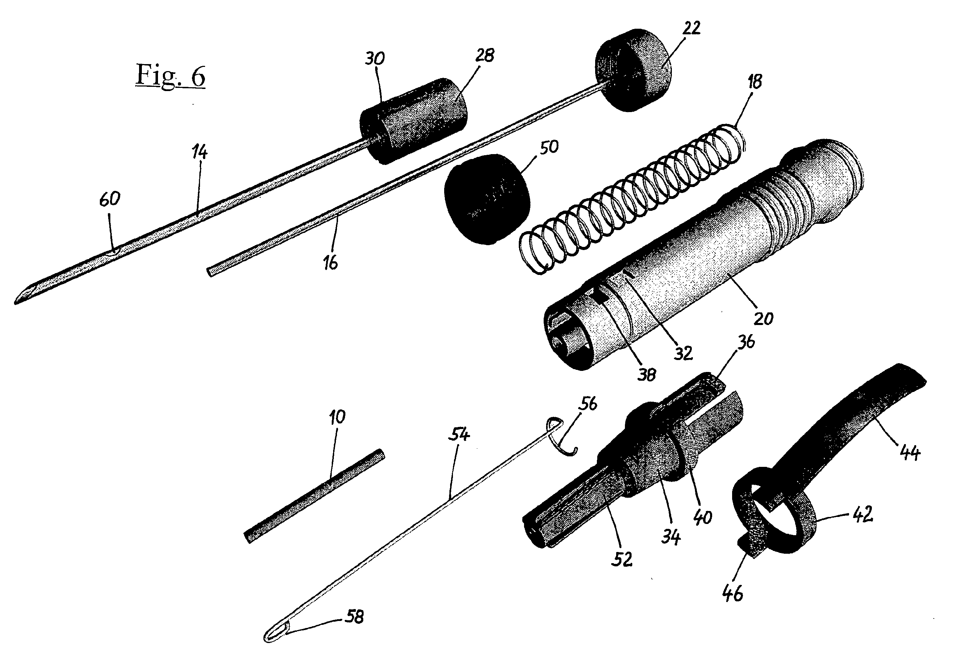

Zu den Einzelheiten der Teile der Implantatspritze wird hauptsächlich auf Fig. 3 und 6 Bezug genommen. Daraus geht hervor, dass der Griff 12 im Ausführungsbeispiel im wesentlichen ein hohlzylindrisches Gehäuse 20 mit einem hinteren, offenen, mit einem Außengewinde versehenen Ende ist, auf das eine Kappe 22 mit Innengewinde aufschraubbar ist, die in ihrem inneren Zentrum mit dem hinteren Ende der Stange 16 verklebt oder in anderer Weise fest verbunden ist.

Mit einem bestimmten Abstand vor dem vorderen Ende ist das hohlzylindrische Gehäuse 20 mit einer Endwand 24 mit einer zentralen, nach vorne weisenden Führungshülse 26 geformt. Die Endwand 24 bildet ein Federlager, an dem sich die als Schraubenfeder ausgebildete Druckfeder 18 mit ihrem vorderen Ende abstützt. Ihr hinteres Ende sitzt auf der radial inneren Wand eines mit einer hinteren Endwand versehenen, doppelwandigen Federkolbens 28, der mit seiner radial äußeren Wand in dem hohlzylindrischen Gehäuse 20 längsverschieblich geführt ist. Durch passende axiale Rippen und Einsenkungen auf der Innenseite des Gehäuses 20 und der Außenseite des Federkolbens 28 ist für eine Verdrehsicherung des Federkolbens 28 in dem Gehäuse 20 gesorgt. Darüber hinaus ist die radial äußere Wand des Federkolbens 28 im vorderen Bereich auf der radial äußeren Seite mit einer Rastnase 30 geformt, die durch Längsschlitze in der Wand des Federkolbens radial elastisch gestaltet ist. Im Ausgangszustand gemäß Fig. 3 greift die Rastnase 30 in ein Loch 32 in der Umfangswand des Gehäuses 20 hinter der Endwand 24 ein. Dadurch wird im Ausgangszustand der Federkolben 28 bei vorgespannter Druckfeder 18 in seiner vorderen Endstellung im Gehäuse 20 verriegelt.

Die Kanüle 14 ist in einer bestimmten Drehwinkelstellung in der zentralen Durchgangsbohrung des Federkolbens 28 fest verklebt oder in anderer Weise befestigt. Sie wird radial gestützt und bei ihrer axialen Bewegung geführt durch die Führungshülse 26. Eine weitere Führung bildet ein am vorderen Ende des Gehäuses 20, vor der Endwand 24 einrastbarer Aufsatz 34, der mit zwei diametral gegenüberliegenden, durch Längsschlitze radial elastisch gestalteten Rastnasen 36 an seinem hinteren, in das Gehäuse 20 einzusteckenden Ende in entsprechende Löcher 38 in der Umfangswand des Gehäuses 20 vor der Endwand 24 einzurasten ist. Auf dem zylindrischen, hinteren Teil des Aufsatzes 34 sitzt zwischen einer äußeren Ringrippe 40 und der Vorderkante des Gehäuses 20 ein Tragring 42 eines Federclips 44, der durch diametral gegenüberliegende axiale Vorsprünge 46 in Aussparungen der Ringrippe 40 verdrehsicher festgelegt ist. Der nach Art eines Federhalter-Clips ausgebildete Federclip 44 ist genau über dem Loch 32 mit einem radial inneren Vorsprung 48 geformt. Somit kann nach dem Einstechen der Kanüle 14 in die Haut und genauer Positionierung der Kanülenspitze und des unmittelbar dahinter gehaltenen Festkörpers 10 die Verriegelung des Federkolbens 28 dadurch gelöst werden, dass radial auf den als Hebel wirkenden Federclip 44 gedrückt wird und dabei die Rastnase 30 nach innen aus dem Loch 32 herausgedrückt wird.

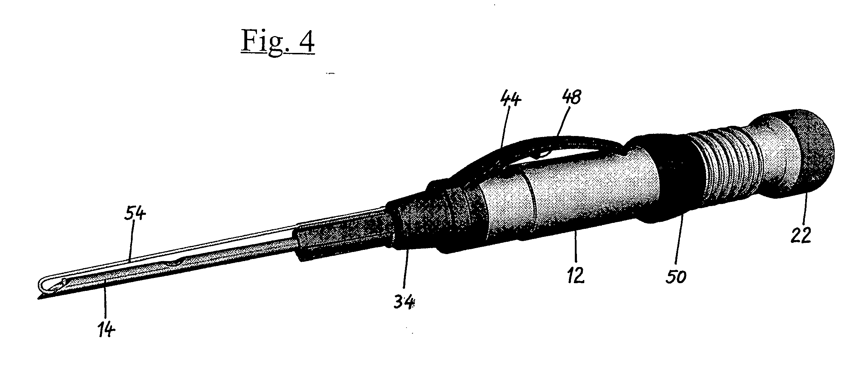

Damit der Rückhub der Kanüle 14 nicht versehentlich schon vor dem Einstechen in die Haut ausgelöst wird, sitzt im Ausgangszustand nach Fig. 1 bis 3 auf der äußeren Umfangsfläche des Gehäuses 20 über dem Loch 32 ein Sicherungsring 50. Er ist mit einer äußeren Ringnut versehen, in die der Vorsprung 48 des Federclips 44 elastisch eingreift. Erst unmittelbar vor der Implantation wird der Sicherungsring 50 nach hinten bis hinter den Federclip 44 in die Stellung nach Fig. 4 verschoben, wobei der Federclip 44 radial nach außen elastisch nachgibt.

Der vordere Bereich des Aufsatzes 34 wird durch einen verjüngten Abschnitt 52 gebildet, der hinten an einem Absatz endet und mit äußeren Längsrippen geformt ist. Auf diesem verjüngten Abschnitt 52 sitzt ein elastischer Draht 54, dessen hinteres Ende 56 zu einem offenen Ring gebogen ist, dessen Innendurchmesser etwas kleiner gewählt ist als der Außendurchmesser des verjüngten Abschnitts 52, dessen Längsrippen etwa im Abstand der Drahtstärke vor dem genannten Absatz enden. Somit kann das hintere Ende 56 des elastischen Drahts 54 axial fixiert zwischen dem Absatz und den Längsrippen auf dem verjüngten Abschnitt 52 sitzen. Wenn der gerade, axial nach vorne gerichtete Hauptteil des Drahts 54 zwischen zwei in Umfangsrichtung im Abstand der Drahtstärke angeordneten Rippen eingelegt wird, kann durch die vorbestimmte Lage der Rippen am Umfang erreicht werden, dass das mit 58 bezeichnete vordere Ende des elastischen Drahts 54, das nach einer Seite von dem Hauptteil des Drahts abgebogen und zu einer Öse umgebogen ist, in die durch das Anspitzen der Kanüle schräg angeschnittene Öffnung ragt. Damit verhindert das vordere Ende des elastischen Drahts 54, dass der Festkörper 10 vor dem Einstechen in die Haut nach vorne aus der Kanüle 14 rutscht. Andererseits stört der elastische Draht 54 beim schrägen Einstechen der Kanüle in die Haut nicht, weil das gerundete vordere Ende 58, auf der Haut gleitend, elastisch ausgelenkt und weggebogen wird. Das den Festkörper 10 in der Kanüle zurückhaltende Sicherungselement 54 könnte auch aus elastischem Kunststoff geformt werden und ist auch bei anderen Implantatspritzen anwendbar, bei denen z. B. in herkömmlicher Weise der Ausstoßer relativ zum Griff axial verschieblich ist.

Ein weiteres Sicherheitselement ist ein Loch 60 in einem bestimmten Abstand hinter der Spitze der Kanüle 14, an einer Stelle, wo sich der Festkörper 10 bestimmungsgemäß befinden muss, wenn er im Ausgangszustand in die Kanüle 14 eingesetzt worden ist. Das Loch 60 gestattet eine einfache visuelle Kontrolle, ob die Implantatspritze zu Beginn des Implantationsvorgangs geladen ist.

Es versteht sich, dass, ausgehend von der gezeigten und beschriebenen Implantatspritze, zahlreiche konstruktive Änderungen möglich sind. So kann z. B. auf die Führung der Kanüle in der mit der Endwand 24 verbundenen Führungshülse 26 verzichtet und der vordere Bereich der Kanüle 14 allein durch eine geeignete Gestaltung des Aufsatzes 32 geführt werden. Diese Konstruktion bietet die Möglichkeit, je nach dem Durchmesser des zu implantierenden Festkörpers 10 Kanülen 14 mit jeweils entsprechenden Durchmessern zu verwenden, ohne am Griff viel ändern zu müssen. Man brauchte im wesentlichen nur den Aufsatz 32 im Innendurchmesser und den Stab 16 im Außendurchmesser anzupassen.

Andererseits könnte, wenn man sich für Implantatspritzen mit jeweils an die unterschiedlich dicken Kanülen angepassten Griffen entscheidet, der Aufsatz 32 einstückig mit dem Gehäuse 20 geformt werden.

Eine weitere Modifikation der Implantatspritze nach Fig. 1 bis 6 besteht darin, dass die Stange 16 nicht am hinteren Ende des Griffs 12 befestigt ist, sondern über einen schmalen, einseitigen Steg an der Endwand 24, der Führungshülse 26 oder dem Aufsatz 32. Diese Konstruktion erlaubt eine wesentlich kürzere Stange 16, bedingt jedoch eine mindestens teilweise längsgeschlitzte Kanüle 14, damit der Steg durch den Längsschlitz hindurchgeführt und mit der Stange 16 verbunden werden kann.

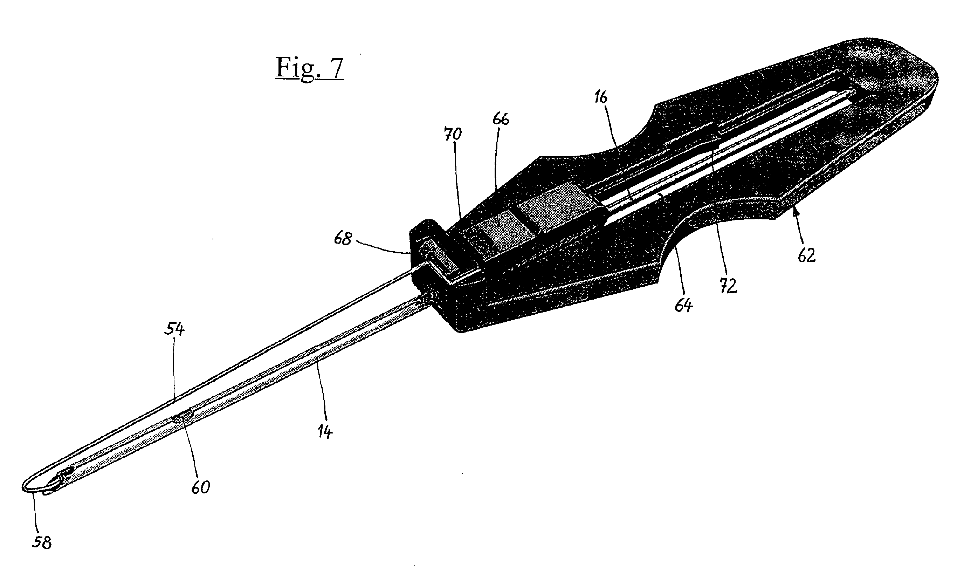

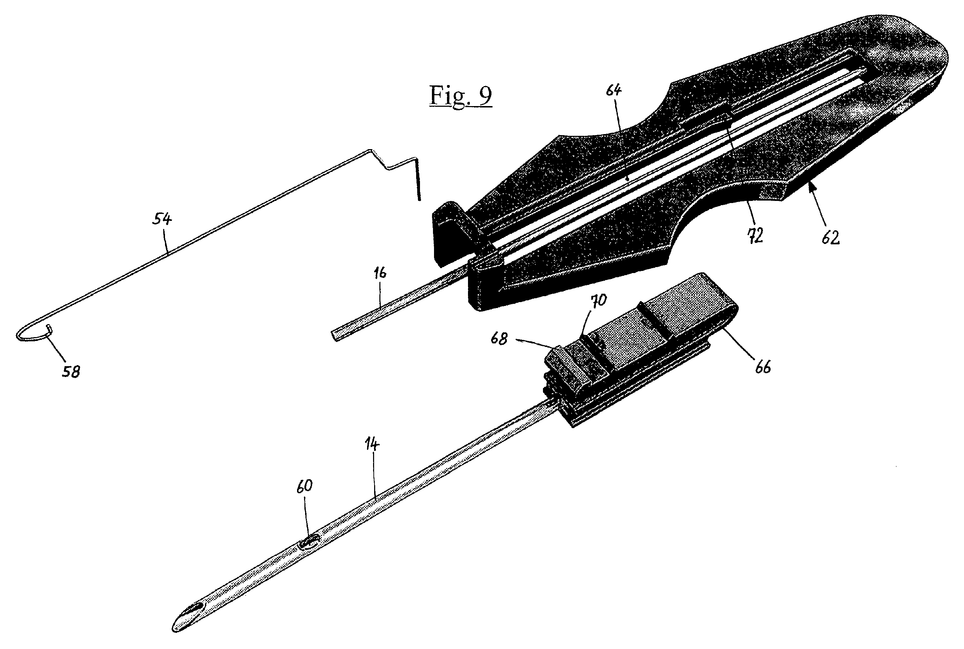

Das Ausführungsbeispiel nach Fig. 7 bis 9 unterscheidet sich funktional dadurch von dem vorstehend beschriebenen Ausführungsbeispiel, dass die auch hier mit 14 bezeichnete Kanüle nach dem Einstechen in die Haut und der vorgesehenen Positionierung des Festkörpers 10 nicht durch eine Feder 18, sondern mit einem Finger der Hand zurückgezogen wird, um den durch die Ausstoßer-Stange 16 axial abgestützten Festkörper 10, eingebettet im Körpergewebe, zu deponieren. Der mit 62 bezeichnete Griff hat im wesentlichen eine flache, längliche, an den Seiten mit griffgünstigen Ausnehmungen versehene und am vorderen Ende portalartig erhöhte Form mit einer sich fluchtend an die Öffnung des Portals anschließenden, sich über den größten Teil der Länge des Griffs 16 erstreckenden Durchbrechung 64 mit seitlichen Führungen für einen mit entsprechenden seitlichen Führungen geformten Schieber 66. Dieser Schieber hat im Längsschnitt die Form eines liegenden U. Sein unterer Schenkel ist mit den seitlichen Führungen geformt und enthält eine zentrale Durchgangsbohrung, in der die Kanüle 14 befestigt ist. Durch die U-Form ist der obere Schenkel in senkrechter Richtung elastisch und an seinem vorderen Ende mit einer Rastnase 68 sowie mit bestimmtem Zwischenabstand dahinter mit einer Rippe 70 geformt. Im Ausgangszustand ist der Sturz des Portals am vorderen Ende des Griffs 62 zwischen der Rastnase 68 und der Rippe 70 eingerastet und dadurch die Kanüle 14 relativ zum Griff 62 fixiert. Zum Zurückziehen der Kanüle 14 von dem an vorbestimmter Stelle unter der Haut deponierten Festkörper 10 wird der obere Schenkel des Schiebers 66 zum unteren Schenkel hin niedergedrückt, um die Verriegelung der Rastnase 68 mit dem Portal zu lösen, und dann in der niedergedrückten Stellung der Rastnase 68 der Schieber 66 längs der Führung in der Durchbrechung 64 zurückgeschoben, bis das vordere Ende der Stange 16 aus der Öffnung der Kanüle 14 herausragt. Vorzugsweise lässt sich die Kanüle 14 sogar bis in eine vollständig in dem Griff 62 aufgenommene Stellung zurückschieben. Um den Schieber 66 in der zurückgeschobenen Stellung zu halten, ist an entsprechender Stelle an der griffseitigen Führung für den Schieber 66 eine seitlich elastisch wegfedernde Rastnase 72 vorgesehen, die hinter der Vorderkante des Schiebers 66 einrastet, wenn dieser vollständig zurückgeschoben wird.

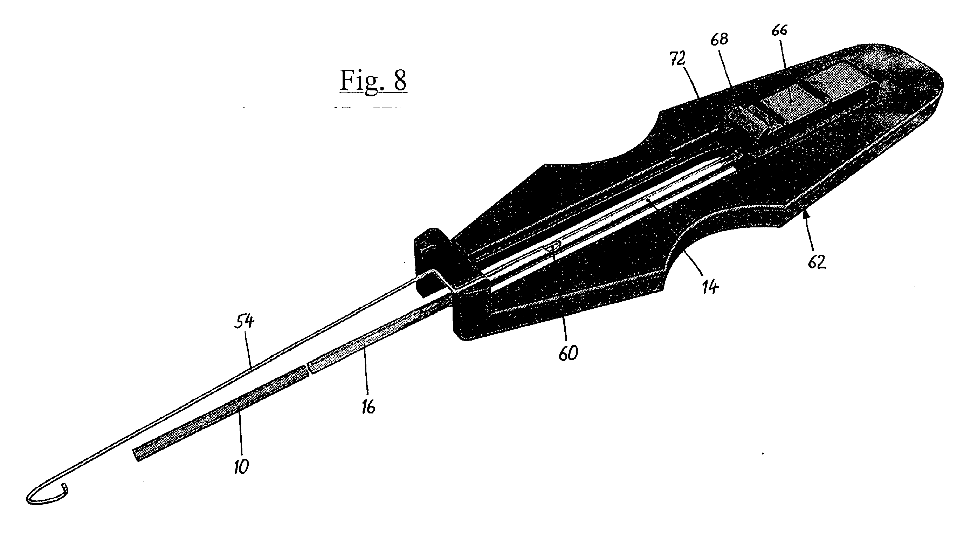

Ebenso wie bei dem zuerst beschriebenen Ausführungsbeispiel ist auch bei der Ausführung gemäß Fig. 7 bis 9 die Stange 16 am hinteren Ende des Griffs 62 befestigt. Sie erstreckt sich durch die Bohrung im Schieber 66 und von hinten in die Kanüle 14 hinein. Letztere ist auch hier mit einem Loch 60 zur Kontrolle des Vorhandenseins eines Festkörpers 10 in der Kanüle 14 versehen. Außerdem wird auch wieder mit einem elastischen Draht 54 mit einem gerundet umgebogenen vorderen Ende 58 gearbeitet, um ein vorzeitiges Herausrutschen des Festkörpers 10 aus der Kanüle 14 zu verhindern. Das hintere Ende des elastischen Drahts 54 ist so gebogen, dass es gemäß Fig. 7 und 8 mit einem sich in Längsrichtung erstreckenden Abschnitt in eine Nut in der Oberseite des Portals am vorderen Ende des Griffs 62 seitlich neben der Portalöffnung einzulegen und mit einem abgewinkelten, sich senkrecht erstreckenden Abschnitt in ein Loch im flachen Teil des Griffs 62 hinter dem Portal einzustecken ist.

Bei der zuletzt beschriebenen Ausführung könnte ebenfalls die Kanüle 14 mit einem Längsschlitz versehen und ein verhältnismäßig kurzer Stab 16 mittels eines sich durch den Längsschlitz erstreckenden Stegs mit dem portalartigen vorderen Ende des Griffs 62 oder hinter dem Portal im Bereich der Führungen mit dem Griff 62 verbunden sein.

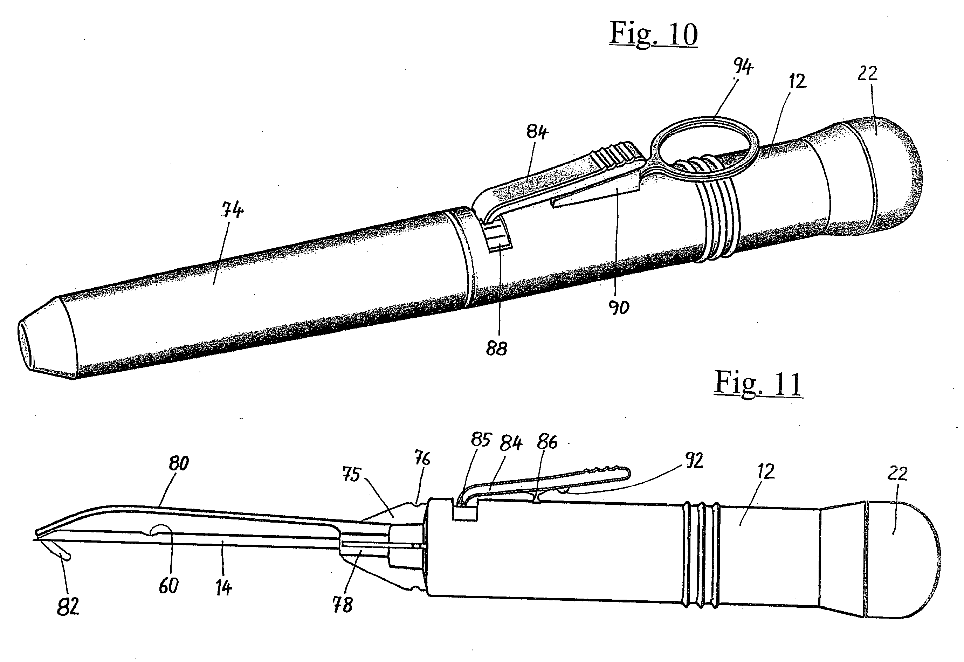

Das Ausführungsbeispiel nach Fig. 10 bis 14 ist ähnlich wie die Ausführung nach Fig. 1 bis 6 gestaltet und funktioniert ebenso wie diese mit einer im Griff 12 aufgenommenen Druckfeder 18, die auf einen Federkolben 28 wirkt und diesen nach dem Lösen einer ihn im Ausgangszustand in einer vorderen Endstellung haltenden Verriegelung im Griff 12 zurückschiebt, wobei die mit dem Federkolben 28 verbundene Kanüle 14 zurückgezogen wird. Insofern, wie Übereinstimmung zwischen den Ausführungen nach Fig. 1 bis 6 und 10 bis 14 besteht, sind die Einzelteile mit denselben Bezugszeichen versehen. Nachstehend braucht nur noch auf Abweichungen eingegangen zu werden.

In Fig. 10 ist eine Schutzhülse 74 gezeigt, die auf das vordere Ende des Griffs aufgeschoben werden kann und im Ausgangszustand die Kanüle 14 überdeckt. Selbstverständlich kann eine entsprechende Schutzhülse auch bei den zuvor beschriebenen Ausführungen nach Fig. 1 und 7 verwendet werden. Wie am besten aus Fig. 11 hervorgeht, ist das vordere Ende des Griffs 12 insgesamt konisch oder mit mehreren sich nach vorne konisch verjüngenden Rippen 75 geformt, die Einsenkungen 76 aufweisen, in welche eine in der Größe passende Ringrippe auf der Innenwand der Schutzhülse 74 nahe bei deren Hinterkante lösbar einrasten kann.

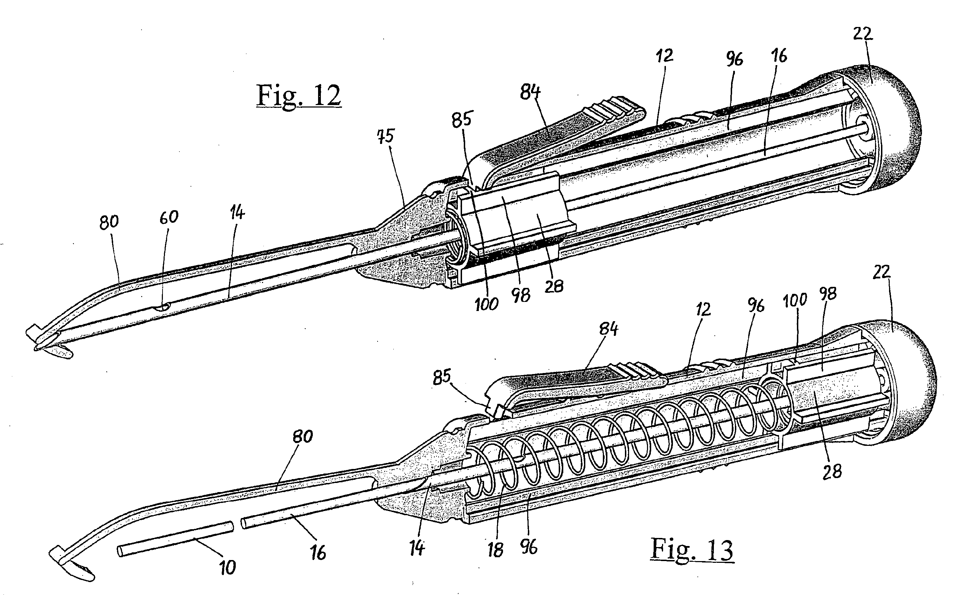

Eine weitere Abweichung von den zuvor beschriebenen Ausführungen besteht darin, dass die vordere Endwand 24 des hohlzylindrischen Griffs 12 einstückig mit dessen Umfangsnwand geformt ist und nach vorne übergeht in eine vorstehende Führungshülse 78, an welche die konischen Rippen 75 und eine weit nach vorne auskragende, elastisch auslenkbare Zunge 80 mit einem hakenförmigen Ende 82 angeformt sind. Die Zunge 80 und das hakenförmige Ende 82 haben dieselbe Funktion wie der Draht 54 und dessen zu einer Öse umgebogenes Ende bei der Ausführung nach Fig. 1 bis 6, d. h. sie sorgen dafür, dass im Ausgangszustand Festkörper 10 nicht aus dem vorne offenen Ende der Kanüle 14 herausfallen können, jedoch beim Ansetzen der Implantatspritze an die Einstichstelle zunächst das hakenförmige Ende 82 der elastischen Zunge 80 auf die Haut trifft, so dass die elastische Zunge 80 ausgelenkt wird und die Öffnung der Kanüle 14 freigibt. Gemäß Fig. 13 ist auch bei dieser Ausführungsform nach dem Zurückziehen der Kanüle 14 deren Öffnung in die Führungshülse 78 des Griffs 12 zurückgezogen.

Darüber hinaus sind die Verriegelung und das Betätigungselement im Vergleich zur Ausführung nach Fig. 1 bis 6 abgewandelt worden. Zur Verriegelung des Federkolbens 28 in seiner vorderen Endstellung und zum Auslösen der Rückzugsbewegung der Kanüle 14 dient ein nach Art einer Wippe ausgebildeter Betätigungshebel 84. Er ist im mittleren Bereich seiner Unterseite durch eine Art Filmscharnier 86 mit der Außenseite der Umfangswand des Griffs 12 verbunden, also einstückig mit diesem ausgebildet. Im Ausgangszustand ist das als Riegel dienende, radial nach einwärts umgebogene vordere Ende 85 des Betätigungshebels 84 niedergedrückt und greift durch ein Loch 88 in der Umfangswand des Griffs 12 hindurch in eine Ausnehmung am Umfang des Federkolbens 28 ein und verriegelt ihn auf diese Weise in seiner vorderen Stellung, in der die Druckfeder 18 komprimiert und die Kanüle 14 nach vorne ausgefahren ist.

Zur Sicherung der Verriegelung des Federkolbens 28 dient ein unter das hintere Ende des Betätigungshebels 84 geschobener Sicherungskeil 90, der durch eine an der Unterseite des hinteren Teils des Betätigungshebels 84 angeformte Rastnase 92, die in eine entsprechende Ausnehmung in der Oberseite des Sicherungskeils 90 eingreift, in der in Fig. 10 gezeigten Stellung gehalten wird. Ein hinten an den Sicherungskeil 90 angeformter Griffring 94 erleichtert das Abziehen des Sicherungskeils 90 bei Ingebrauchnahme der Implantatspritze. Nach dem Abziehen der Schutzhülse 74 und des Sicherungskeils 90 kann die Implantatspritze an der vorgesehenen Einstichstelle der Haut angesetzt und eingestochen werden. Wenn dann anschließend das hintere Ende des Betätigungshebels 84 niedergedrückt wird, wobei dieser um das Filmscharnier 86 verschwenkt, wird das vordere Ende 85 des Betätigungshebels 84 radial nach außen aus der Eingriffsstellung mit dem Federkolben 28 zurückgezogen und dadurch entriegelt, so dass er durch die gespannte Druckfeder 18 im Griff 12 zurückgeschoben werden kann und dabei die Kanüle 14 mitnimmt.

Aus Fig. 12, 13 und 14 ist ersichtlich, dass die Umfangswand des Griffs 12 mit sich über ihre Länge erstreckenden, radial nach innen abstehenden Rippen 96 geformt ist. Der Federkolben 28 ist ebenfalls mit axialen Rippen 98 geformt, die radial nach außen abstehen. Mindestens eine der Rippen 96 hat im Zusammenwirken mit den Rippen 98 die Funktion, eine Drehung des Federkolbens 28 um seine Mittelängsachse zu verhindern, so dass das Kontroll-Loch 60 immer auf der Oberseite der Kanüle 14 sichtbar bleibt. Außerdem haben die inneren Längsrippen 96 im Griff 12 eine Führungsfunktion. Sie sorgen nämlich dafür, dass die Druckfeder 18 auch unter Druckspannung gerade bzw. konzentrisch bleibt und sich bei der Montage nicht verwindet oder knickt. Drittens schließlich bewirken die den Federkolben 28 führenden Rippen 96 und 98 bei der Verschiebung des Federkolbens 28 durch die Druckfeder 18 einen geringen Verschiebe-Widerstand, da während dieser Bewegung dass im hinteren Bereich des inneren Hohlraums des Griffs 12 befindliche Luftvolumen ohne weiteres an dem nach hinten verfahrenden Federkolben 28 vorbei in den vorderen Bereich des Griffs verdrängt werden kann.

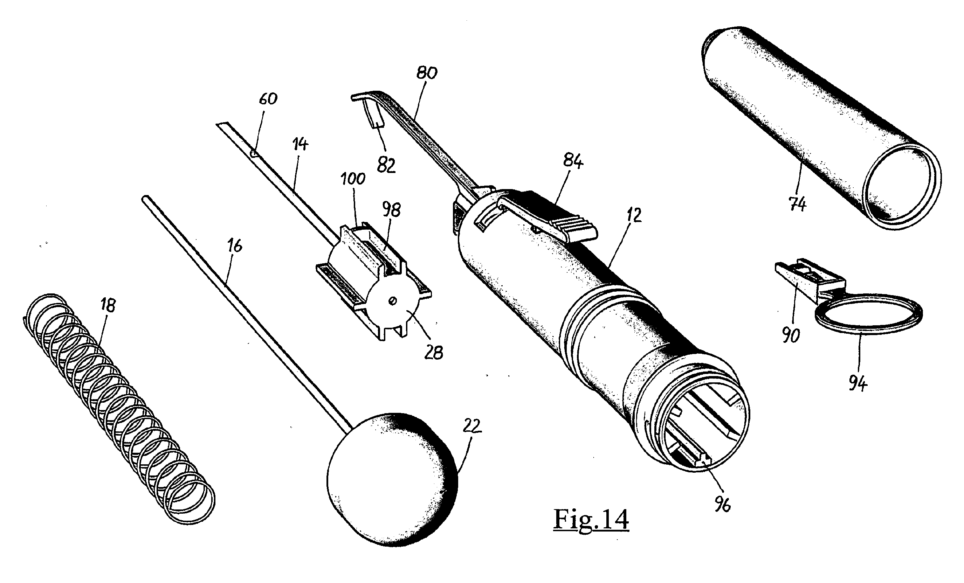

Gemäß Fig. 14 sind die beiden - ebenso wie das Loch 60 - nach oben weisenden Rippen 98 durch eine Querrippe 100 verbunden. Auf diese Weise bilden die drei miteinander verbundenen Rippen 98, 100 eine nach oben offene Ausnehmung, in die das als Riegel dienende vordere Ende 85 des Betätigungshebels 84 eingreift, um in Anlage an der Rückseite der Querrippe 100 den Federkolben 28 in seiner vorderen Ausgangsstellung zu verriegeln.

## Patentansprüche

1. Implantatspritze mit einer an einem Griff (12, 62) axial verschieblich gelagerten Kanüle (14) und einem in dieser angeordneten Ausstoßer (16) für die transkutane Einführung von z. B. Arznei- oder Schmerzmittel enthaltenden Festkörpern (10) durch die Kanüle (14) in den menschlichen oder tierischen Körper, wobei die Kanüle (14) und der Ausstoßer (16) in einer Relativstellung am Griff (12) festlegbar sind, in der sich das vordere Ende des Ausstoßers (16) in einem bestimmten, für die Aufnahme eines oder mehrerer Festkörper (10) in der Kanüle (14) ausreichenden Abstand von deren Spitze befindet, und dass die Kanüle (14) nach dem Lösen einer Sperre (30, 32; 68; 84, 100) relativ zum Griff (12) und dem Ausstoßer (16) bis hinter dessen vorderes Ende zurückziehbar ist, **dadurch gekennzeichnet, dass** die Kanüle (14) mittels eines in einem hohlzylindrischen Griff (12) axial geführten Federkolbens (28) durch die Kraft einer in dem zylindrischen Hohlraum des Griffs (12) aufgenommenen, vorgespannten Feder (18) zurückziehbar ist.

2. Implantatspritze nach Anspruch 1, **dadurch gekennzeichnet, dass** die Spitze der Kanüle (14) bis in eine geschützte Lage im Griff (12, 62) zurückziehbar ist.

3. Implantatspritze nach Anspruch 1 oder 2, **dadurch gekennzeichnet, dass** die Kanüle (14) von einer Schrauben-Druckfeder (18) umgeben ist, die im vorgespannten Zustand in dem zur Kanüle (14) koaxialen, zylindrischen Hohlraum im Griff (12) zwischen einem Federlager (24) und dem mit dem hinteren Ende der Kanüle verbundenen Federkolben (28) eingespannt ist.

4. Implantatspritze nach einem der Ansprüche 1 bis 3, **dadurch gekennzeichnet, dass** bei vorgespannter Feder (18) der Federkolben (28) durch einen Riegel (30, 85) am Griff (12) festlegbar und mittels eines an der Außenseite des Griffs angebrachten, auf den Riegel (30, 85) wirkenden Betätigungselements (44, 48, 84) entriegelbar ist.

5. Implantatspritze nach Anspruch 4, **dadurch gekennzeichnet, dass** der Riegel (30,) durch eine radial beweglich am Griff (12) oder Federkolben (28) angebrachte Nase (85) gebildet ist, die im verriegelten Zustand in eine Ausnehmung (32, 98, 100) in dem Federkolben (28) bzw. in der Umfangswand des zylindrischen Hohlraums im Griff (12) eingreift.

6. Implantatspritze nach Anspruch 4 oder 5, **dadurch gekennzeichnet, dass** der verriegelte Zustand des Federkolbens (28) durch ein auf der Außenseite des Griffs (12, 20) in einer Sperrstellung lösbar festlegbares, das Betätigungselement (44, 48, 84) blockierendes Sicherungsglied (50, 90) gesichert ist.

7. Implantatspritze nach Anspruch 6, **dadurch gekennzeichnet, dass** das Betätigungselement (44, 84) nach Art eines Federhalter-Clips ausgebildet ist und einen zum Griff (12, 20) weisenden Vorsprung (48, 85) hat, der als zurückziehbarer Rigel in die Ausnehmung (98, 100) in dem Federkolben (28) eingreift oder radial in Löserichtung gegen eine in eine Ausnehmung (32) im Griff (12) eingreifende Rastnase (30) am Federkolben (28) andrückbar ist.

8. Implantatspritze nach einem der Ansprüche 3 bis7, **dadurch gekennzeichnet, dass** der äußere Umfang des Federkolbens (28) und die innere Umfangsfläche des hohlzylindrischen Griffs (12) derart mit sich axial erstreckenden Rippen (96, 98) geformt sind, dass der in einer bestimmten relativen Drehstellung in den Griff eingesetzte Federkolben (28) gegen Drehung und die Feder (18) gegen seitliche Auslenkung gehalten ist.

9. Implantatspritze nach einem der Ansprüche 1 bis 8, **dadurch gekennzeichnet, dass** der Ausstoßer (16) mit seinem hinteren Ende ein am hinteren Ende des Griffs (12, 20) befestigter, sich durch den Federkolben (28) in die Kanüle (14) erstreckender Stab ist, dessen vorderes Ende aus dem vorderen, verjüngten Ende (34) des Griffs (12, 20) herausragt.

10. Implantatspritze nach einem der Ansprüche 1 bis 8, **dadurch gekennzeichnet, dass** der Ausstoßer (16) ein am vorderen Ende des Griffs (12, 20; 62) über einen einseitig angeordneten, schmalen, radialen Steg befestigter Stab ist und die Kanüle (14) mit einem Längsschlitz versehen ist, durch den sich der radiale Steg erstreckt.

11. Implantatspritze nach dem Oberbegriff des Anspruchs 1, **dadurch gekennzeichnet, dass** das hintere Ende der Kanüle (14) mit einem längsverschieblich am Griff (62) geführten Schieber (66) verbunden ist, der in der vorderen Endstellung durch einen Riegel (68) am Griff (62) festlegbar und nach dem Lösen der Verriegelung mit einem Finger zurückziehbar ist.

12. Implantatspritze, insbesondere nach einem der Ansprüche 1 bis 9, **dadurch gekennzeichnet, dass** am vorderen Ende des Griffs (12, 20, 34; 62; 78) ein elastischer Draht (54) befestigt oder eine elastische Zunge (80) aus Kunststoff angebracht oder angeformt ist, dessen bzw. deren zu einer Öse, einem Haken oder einem Kopf gebogenes freies Ende (58, 82) sich unmittelbar vor der Öffnung der in ihre vordere Endstellung vorgeschobenen Kanüle (14) befindet und beim Einstechen der Kanüle (14) in die Haut auslenkbar ist.
